# EUROPEAN PATENT SPECIFICATION

(11) **EP 1 205 543 B1**
(45) Date of publication and mention of the grant of the patent: **02.03.2005**
(21) Application number: 00203911.3
(22) Date of filing: 09.11.2000
(51) Int. Cl.: C12N 9/12, C12N 15/82, C12N 15/63

(54) **DNA sequences encoding proteins conferring Phytophthora infestans resistance on plants**
DNA Sequenzen, die Proteine kodieren, welche Resistenz gegen Phytophthora infestans in Pflanzen vermitteln
Séquences d'ADN codant des protéines, pour conférer aux plantes une résistence contre Phytophthora infestans

(43) Date of publication of application: 15.05.2002
(73) Proprietor: Plant Research International B.V., 6708 PB Wageningen (NL)
(72) Inventor: Van Enckevort, Leonora Johanna Gertruda, 6702 BD Wageningen (NL); Jacobsen, Evert, 6706 BD Wageningen (NL); Stiekema, Willem Johannes, 6708 NB Wageningen (NL); Pereira, Andy, 6716 GG Ede (NL)
(74) Representative: De Hoop, Eric

(56) References cited:
- VAN ENCKEVORT L.J.G. ET AL.: "Selection of independent Ds transposon insertions in somatic tissue of potato by protoplast regeneration" THEORETICAL AND APPLIED GENETICS, vol. 101, September 2000 (2000-09), pages 503-510, XP000926420
- EL-KHARBOTLY A. ET AL.: "Localization of Ds-transposon containing T-DNA inserts in the diploid transgenic potato: linkage to the R1 resistance gene against Phytophthora infestans (Mont.) de Bary" GENOME, vol. 39, no. 2, April 1996 (1996-04), pages 249-257, XP000926428
- TARCHINI R. ET AL.: "The Complete Sequence of 340 kb of DNA around the Rice Adh1-Adh2 Region Reveals Interrupted Colinearity with Maize Chromosome 4" THE PLANT CELL, vol. 12, no. 3, March 2000 (2000-03), pages 381-391, XP002168485
- KANEKO T. ET AL.: "Structural analysis of Arabidopsis thaliana chromosome 5.V. Sequence features of the regions of 1,381,565 bp covered by twenty one physically assigned P1 and TAC clones" DNA RESEARCH , vol. 5, no. 1, 30 April 1998 (1998-04-30), pages 131-145, XP000926429
- ALCALA J. ET AL.: "Generation of ESTs from tomato carpel tissue; EST267901 tomato ovary, TAMU Lycopersicon esculentum cDNA clone cLED34E19, mRNA sequence" EMBL DATABASE ENTRY AI898458; ACCESSION NO. AI898458, 28 July 1999 (1999-07-28), XP002168486
- SUNDARESAN V. ET AL.: "PATTERNS OF GENE ACTION IN PLANT DEVELOPMENT REVEALED BY ENHANCER TRAP AND GENE TRAP TRANSPOSABLE ELEMENTS" GENES AND DEVELOPMENT, vol. 9, no. 14, 15 July 1995 (1995-07-15), pages 1797-1810, XP000674520 ISSN: 0890-9369
- MEISSNER R. ET AL.: "A new model system for tomato genetics" THE PLANT JOURNAL, vol. 12, no. 6, 1997, pages 1465-1472, XP002096585 ISSN: 0960-7412

## Description

### FIELD OF THE INVENTION

This invention relates to methods and materials for improved plant disease resistance. In particular the present invention relates to nucleic acid sequences required for resistance of potato to *Phytophthora infestans*, recombinant polynucleotide molecules containing the sequences, and uses thereof to transform plants, especially plants of the family *Solanaceae* to make them more resistant to *Phytophthora* species.

### BACKGROUND OF THE INVENTION

The oomycete pathogen *Phytophthora infestans*, is worldwide the main disease of the potato crop causing late blight that results in major losses of crop yield and quality. *P. infestans* infects plants of commercial importance like potato and tomato, that therefore require regular chemical control. Monogenic *R* genes have been introduced from the hexaploid Mexican wild species *Solanum demissum* into the cultivated tetraploid potato cultivars (Wastie, 1991). These race specific *R* genes did not provide durable field resistance because of the rapid evolution of new virulent races of the fungus that circumvent these *R* gene mediated resistances. Characteristic for *R* gene mediated resistance reactions is the hypersensitive response (HR) leading to local cell death causing necrotic spots at the site of attempted infection. Genetic analysis showed that activation of HR is highly specific and induced upon recognition by a specific *R* gene product and a corresponding avirulence gene product in the pathogen (Hammond-Kosack and Jones, 1997).

The *R* gene mediated resistance from wild *Solanum* species can show partial resistance or an intermediate HR response when crossed to different *S. tuberosum* backgrounds (Graham, 1963; Toxopeus, 1958). The HR lesions can vary in size depending on the backcross parent used, indicating that other genes influence the *R* gene resistance reaction. Minor *S. tuberosum* or *S. demissum* genes have been characterized to influence or even suppress *R* gene expression (El-Kharbotly *et al*., 1996b). QTL mapping in *S*. *tuberosum* populations segregating for partial *P. infestans* resistance, identified 19 QTLs on 13 chromosomal regions (Leonards-Schippers *et al.,* 1994), with one QTL on chromosome 5 near the *P. infestans* resistance locus *R1* also linked to QTLs for maturity and vigor (Collins *et al*., 1999). These QTLs on chromosome 5 very likely represent minor genes that play a role in both *R* gene mediated HR resistance responses and developmental processes which indirectly influence the resistance response. Additionally, this chromosome region also contains several other resistance loci with specificity to different pathogens like the PVX virus (Ritter *et al*., 1991) and potato cyst nematodes (Kreike *et al*., 1994; Rouppe van der Voort *et al*., 1998).

The cloning of *R* genes that mediate gene-for-gene type resistance to bacterial, fungal, oomycete, viral, and nematode pathogens has so far identified 5 classes of genes based on common characteristics including nucleotide binding sites, leucine-rich repeats, transmembrane domains and serine/threonine protein kinases (Hammond-Kosack and Jones, 1997). Genetic mapping and sequence analysis showed frequent clustering of *R* genes with different resistance specificities at complex loci (Jia *et al*., 1997; Parniske *et al*., 1997). Despite these insights into *R* gene structure their function can not be predicted from sequence alone and functional tests are required to determine their role in resistance (Parker *et al*., 1996).

A few *R* gene signal transduction components have been identified by mutation (reviewed in Innes, 1998). These analyses have helped identify genes that are required for the barley powdery mildew mediated *Mla-12* resistance (*rar-1* and *rar-2*; Jørgensen, 1996), the tomato *Pseudomonas syringae* pv *tomato* resistance gene *Pto* (*Prf*; Salmeron *et al*., 1996) and *Pti;* (Zhou *et al*., 1995) and for the tomato *Cf-9* (*rcr-1* and *rcr-2;* Hammond-Kosack and Jones, 1994) and *Cf-2* mediated *Cladosporium fulvum* resistance reactions (*rcr-3;* Jones *et al*., 1999). Extensive mutant screens in Arabidopsis identified a number of genes involved in plant pathogen interactions, *ndr1* (Century *et al*., 1995), *eds1* (Parker *et al*., 1996), *pad1, pad2, pad3* and *pad4* (Glazebrook *et al.,* 1996) and *pbs1*, *pbs2 and pbs3* (Warren *et* *al*., 1999). Most of these mutations affect the function of a subset *of R* genes (Aarts *et al*., 1998) or only combinations of double mutations significantly decrease *R* gene resistance (Glazebrook *et al*., 1997; Warren *et al*., 1999; McDowell *et al*., 2000). This indicates the occurrence of different signaling pathways for resistance reactions that are also partially redundant.

Transposon tagging is an established tool in plants for the identification of genes that display a mutant phenotype when their function is disrupted. Transposons have been introduced from maize and successfully used for tagging in many heterologous plants like Arabidopsis (Aarts *et al*., 1993), petunia (Chuck *et al*., 1993), tobacco (Whitham *et al*., 1994), tomato (Jones *et al*., 1994) and flax (Lawrence *et al*., 1995). In these self-fertilizing plant species random tagging strategies (Arabidopsis, petunia) by screening large selfed populations for mutants or targeted tagging of specific genes (tobacco, tomato, flax) were applied. By self- or test- crossing, large populations were produced for the direct screening of possible transposon tagged mutants. By using selectable markers like kanamycin (Baker *et al*., 1987) or hygromycin (Rommens *et al*., 1992), selection of excision events at the cellular level has been feasible and in combination with effective in vitro selection and somatic propagation procedures can facilitate the production of large numbers of transposon insertion mutants.

### SUMMARY OF THE INVENTION

The present invention provides an isolated DNA sequence which encodes a protein having the amino acid sequence given in SEQ ID NO:2 or a functionally homologous protein having an amino acid sequence showing an identity of at least 80% to SEQ ID NO:2, which protein confers *Phytophthora infestans* resistance on plants.

Preferably, the DNA sequence encodes a protein having an amino acid sequence showing an identity of at least 85%, or even 90%, to SEQ ID NO:2.

More preferably, the DNA sequence encodes the amino acid sequence given in SEQ ID NO:2, in which case the DNA sequence comprises the nucleotide sequence given in SEQ ID NO: 1.

More general, the invention provides the DNA sequence selected from the group consisting of:
a) the DNA sequence given in SEQ ID NO: 1 and its complementary strand, and
b) DNA sequences hybridizing to the sequences in (a) under stringent hybridization conditions.

In particular the DNA sequence comprises the nucleotide sequence of nucleotides 20 to 1053 of SEQ ID NO: 1, which is the coding sequence for the protein having the amino acid sequence given in SEQ ID NO:2. Said nucleotide sequence contains an intron, nucleotides 584 to 726. Accordingly, the actual DNA sequence encoding the protein of SEQ ID NO:2 comprises nucleotides 20 to 583 and 727 to 1053 of SEQ ID NO:1.

In a further aspect the invention provides a protein having the amino acid sequence given in SEQ ID NO:2 or a functionally homologous protein having an amino acid sequence showing an identity of at least 80% to SEQ ID NO:2, which protein confers *Phytophthora infestans* resistance on plants.

In another aspect the invention provides a recombinant vector comprising a DNA sequence as defined above under control of an appropriate promoter and regulatory elements for expression in a host cell.

In still another aspect the invention discloses the use of the present DNA sequence or recombinant vector for the production of a transgenic plant.

Further the invention provides a host cell, preferably a plant cell, comprising the present DNA sequence or recombinant vector.

Also provided is a plant or any part thereof comprising such plant cell, and seed, selfed or hybrid progeny or descendant of such a plant, or any part thereof.

The invention also provides a method of conferring *Phytophthora infestans* resistance on a plant, comprising the steps of
i) introducing a DNA sequence as defined above or a recombinant vector as defined above into a cell of the plant or an ancestor thereof,
ii) regenerating plants from the obtained transgenic cells, and
iii) selecting plants exhibiting *P. infestans* resistance.

A further aspect of the invention is the provision of oligonucleotide probes that comprise a sequence of nucleotides of SEQ ID 1, or a mutant, derivative or allele thereof, capable of detecting the pathogen resistance gene or functional equivalents thereof in plants of the family *Solanaceae* and the use of the probes to isolate DNA sequences encoding a pathogen resistance gene or a functional equivalent thereof.

Using the sequence SEQ ID 1 facilitates the isolation of homologous genes from related and unrelated hosts to obtain genes, which protect host plants against related and unrelated pathogens.

A further aspect of the invention is the identification of proteins that interact with constructs comprising sufficient homology to SEQ ID 2, the genes thereof can be used to provide plant cells that are resistant to pathogens. One way is by identification of interacting proteins by the yeast two-hybrid system that are then involved in the signal transduction of the resistance response.

A further aspect of the invention is the construction of hybrid proteins comprising SEQ ID 2 or DNA isolates of sufficient homology, with other proteins that can be used as effector molecules. One way is by making hybrids with different leucine rich repeat fragments from various plants or synthetically produced *in vitro,* that can interact with different pathogen or inducer effector molecules. These effectors can also be chemically produced and by application to a plant containing the hybrid construct can induce the signal transduction pathway for resistance.

### BRIEF DESCRIPTION OF THE DRAWINGS

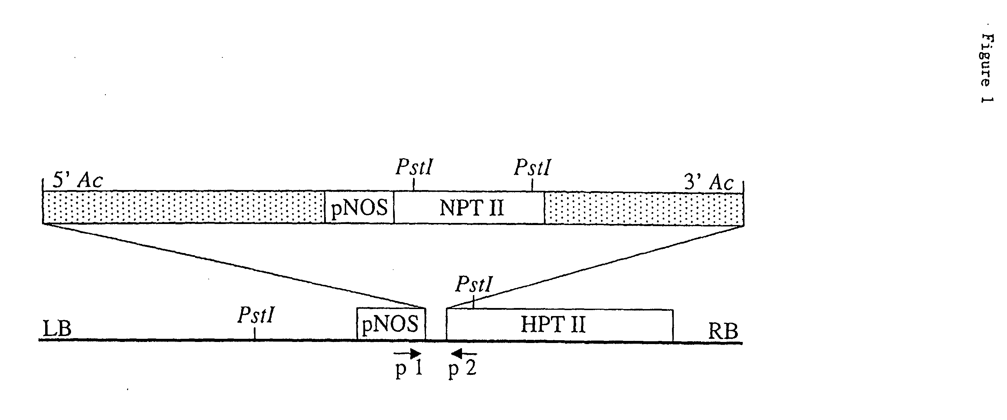

Figure 1: Schematic drawing of pHPT::*Ds*-Kan showing positions of primer 1 (p1, GCG CGT TCA AAA GTC GCC TA), primer 2 (p2, GTC AAG CAC TTC CGG AAT CG) and *Pst1* restriction sites. Abbreviations: LB = left border, RB = right border, pNOS = nopaline synthase promoter, NPT II = neomycin phosphotransferase gene, HPT II = hygromycin phosphotransferase gene.

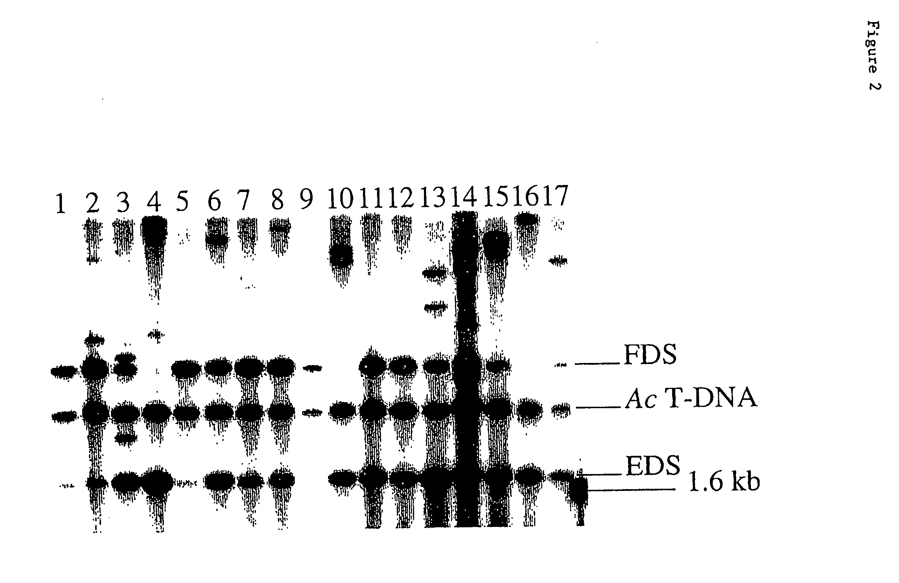

Figure 2: *Pst1* restriction of genomic DNA hybridized to NOS promoter probe to select for presence of full donor site (FDS = 4.0 kb), empty donor site (EDS = 2.3 kb), *Ac* T-DNA construct (3.5 kb) and *Ds* re-insertion sites in the RIDs/r-; Ac/- selected seedlings EE96-4311-37 (lane 1), EE96-4312-43 (lane 5) EE96-4312-49 (lane 9) and HygR protoplast regenerants from EE96-4311-37 (lane 2,3 and 4), EE96-4312-43 (lane 6, 7 and 8), EE96-4312-49 (lane 10, 11, 12 and 13), EE96-4311-15 (lane 13, 15), EE96-4312-05 (lane 14), EE96-4312-76 (lane 16) and EE96-4312-06 (lane 17).

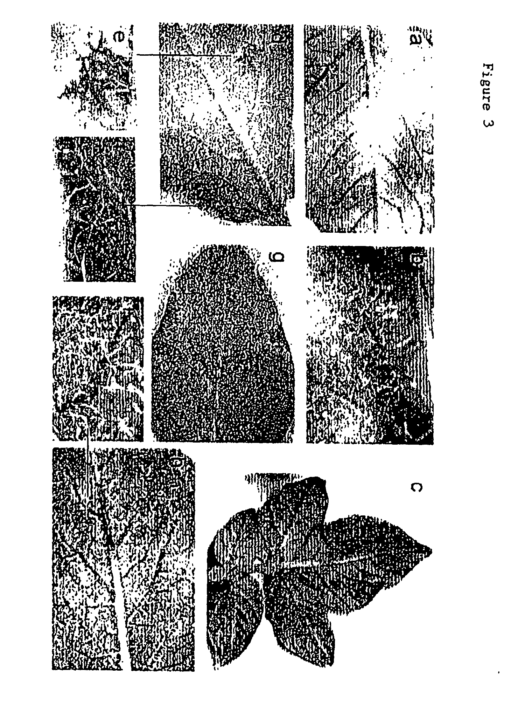

Figure 3: Reaction phenotypes observed on different genotypes after inoculation of detached leaves with *P*. *infestans* race 0. a) TM17-2, susceptible parent; b) detail of sporulation on TM17-2; c) HRPR 836; d) HRPR 1587 showing both the *R1* type HR response and necrotic regions with sporulation; e) detail of HR spot on HRPR 1587; f) detail of sporulation on the necrotic region of HRPR 1587; g) necrotic regions on RlDs/r-; Ac/- seedling EE96-4312-03, minor sporulation was detected in such regions; h) clear colonization on variant 1000; i) detail of sporulation on variant 1000.

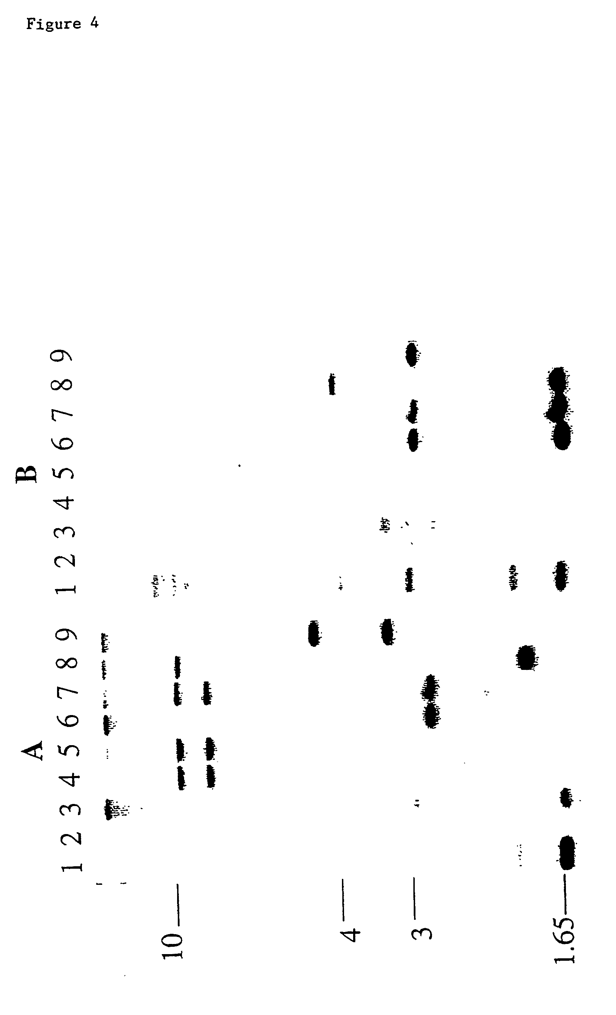

Figure 4: *Hin*dIII digested genomic DNA hybridized to the 5' Ac probe (A) or the internal *Ac* probe (B). Lane 1 shows the 1.6-kb marker hybridization. The *R1* resistant crossing parent J91-6167-2 (lane 2a and b) and the susceptible crossing parent 87-1024-2 (lane 3a and b) contain both no *Ac* or *Ds* elements. The primary transformant Ds416 contained two *Ds* T-DNA loci (lane 4a), Ds53-34 inherited both *Ds* T-DNA loci (lane 5a) as did EE96-4312-28 (lane 7a). EE96-4312-28 inherited from TM17-2 (lane 6a) the *Ac* element. In mutant 487 (lane 8a) and mutant 1000 (lane 9b), both regenerated from EE96-4312-28, the *Ds* elements transposed to new positions and *Ac* seems to be missing. In TM17-2 (lane 6b) a complete *Ac* (1.6 kb internal HindIII fragment) and a d4c (2.9 kb) are present. Mutant 487 (lane 8b) inherited d*Ac* as a different restriction fragment due to the insertion of *Ac* in d*Ac*. In mutant 1000 (lane 9b) Ac got lost and only dAc is present.

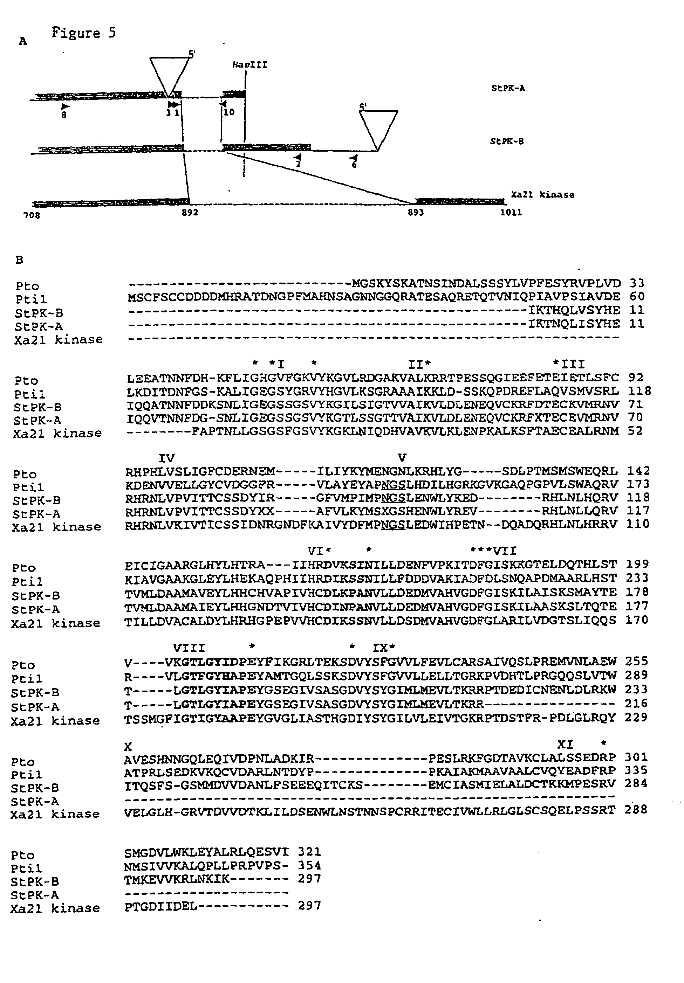

Figure 5: a) Schematic representation of the isolated *Ds* flanking sequences from mutant 1000 (*rpr1* and *rpr2*) and their alignment to XA21 aa 708- 1011. The large triangles represent the *Ds* positions, the intron region is a dashed line and the small black triangles represent primer positions of EE1 (5'-ACA TTG GGC ACT CTT GGA TAC A), EE2 (5'- TCT TGA TTC TGG CAT TTT CTT TG), EE3 (5'- CCT GAC ACA AAC CGA GAC ATT), EE6 (5'- AAC AAT GCC TTT CTT CTC), EE8 (5'-GCA CAT TAT CAA GTG GAA CTA CG) and EE10 (5'- CTG AGC CGT ACT CTT AAA AGA ACG). b) Amino acid alignment of Pto, Pti1, StPK-B, StPK-A and Xa21. The eleven conserved domains of a protein kinases are numbered and the conserved amino acids are marked (*). Bold domains are specific for serine/threonine recognition. The N-glycosylation site is underlined.

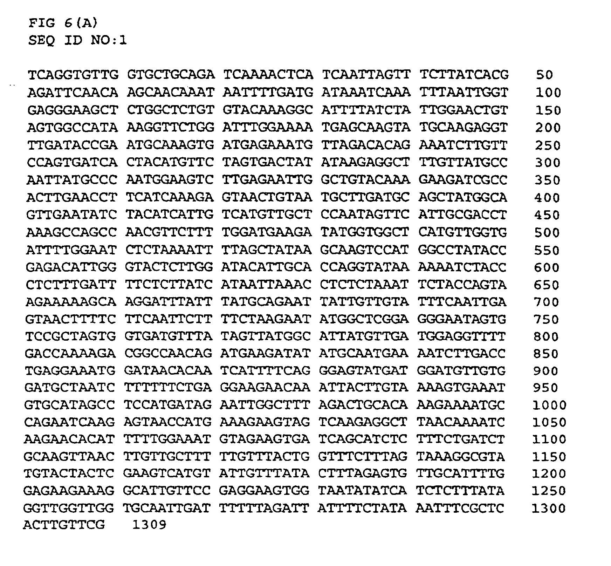

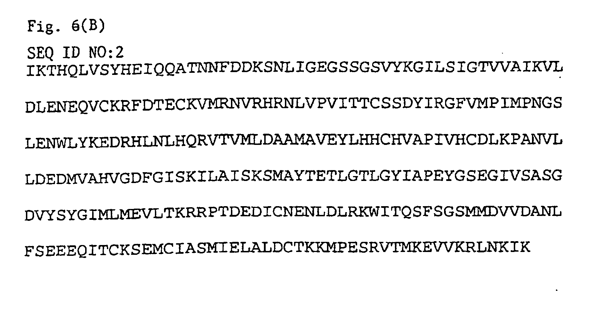

Figure 6 (A and B): Sequence of the StPK-B DNA (SEQ ID NO: 1) and StPK-B protein (SEQ ID NO: 2). StPK-B DNA sequence is the contiguous sequence of a DNA fragment obtained by isolating the Ds tagged gene. The sequence is part of the StPK-B gene encoding the protein kinase domain and lacks the N-terminal portion of the gene including the translation start. SEQ ID 2 is the amino acid translation of SEQ ID 1 after removing the predicted intron conserved with other gene family members.

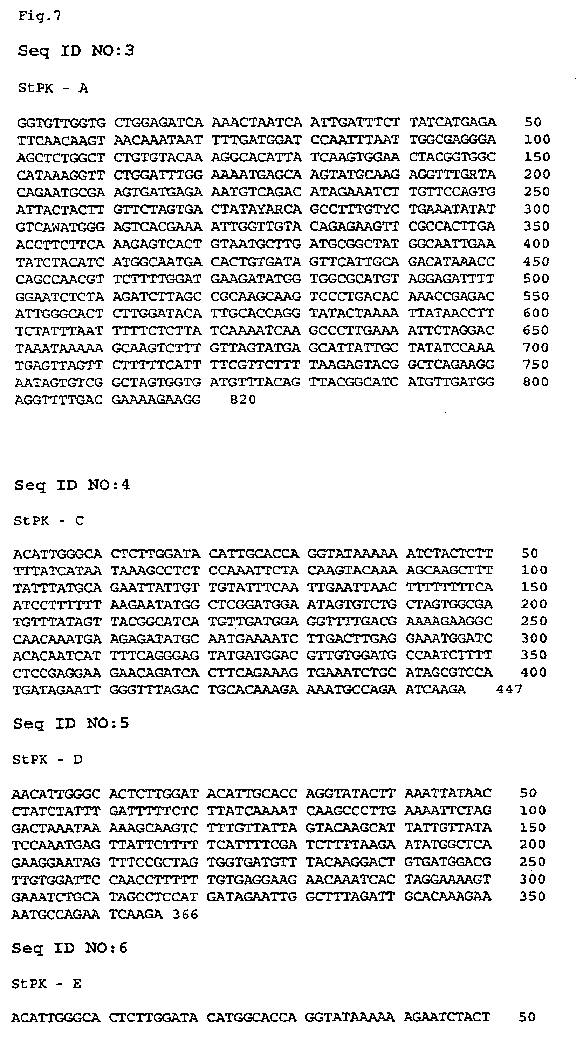

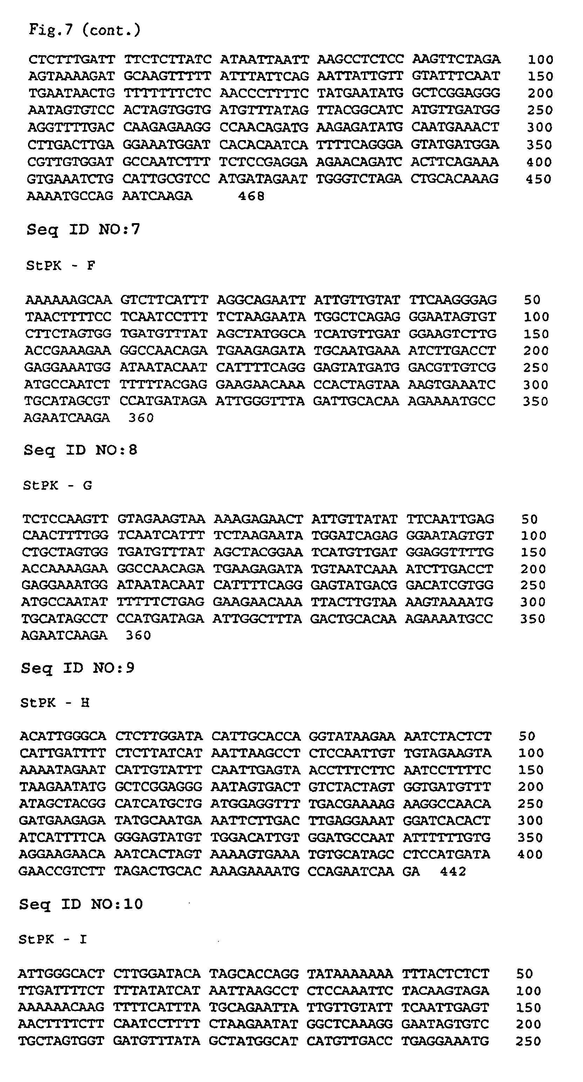

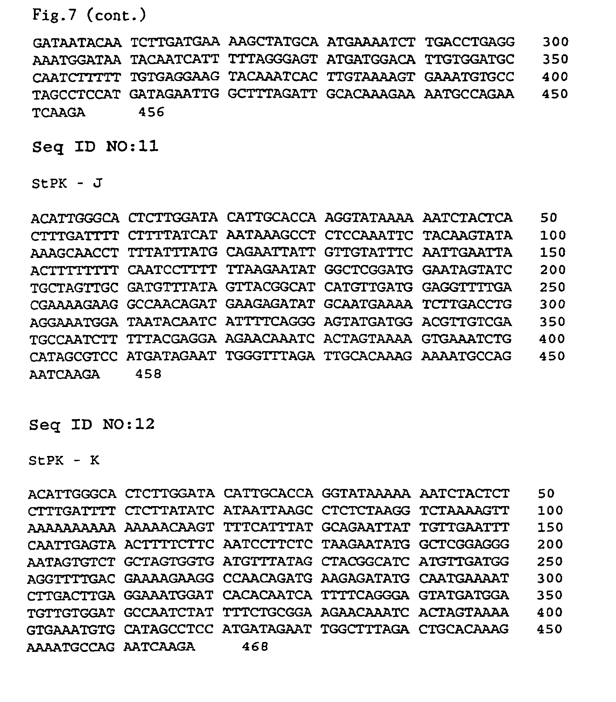

Figure 7: DNA sequences of StPK homologs obtained by using primers EE1 and EE2. SEQ ID 3 is the sequence of DNA fragments of StPK-A that is tagged by the transposon Ds. SEQ ID 4-12 are DNA fragments obtained by PCR between primers EE1 and EE2; Seq ID 4-12 represent fragments of homologous sequences StPK-C to StPK-K, in the genome.

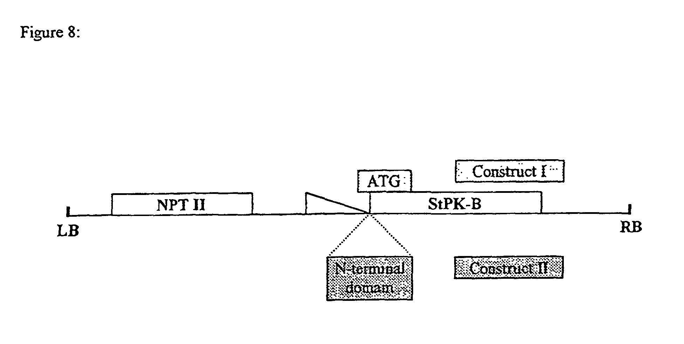

Figure 8: Diagram of StPK overexpression constructs used for plant transformation. The StPK-B gene with a synthetic translation initiation start, under control of the CaMV 35S promoter and Nos-terminator is cloned in a binary vector for transformation of plants using Agrobacterium tumefaciens strains in construct I. Construct II contains the N-terminal part of the StPK genes or other N-terminal fusions to protein domains that act as recognition domains with other effector molecules.

### DETAILED DESCRIPTION OF THE INVENTION AND EXPERIMENTS

### Development of transposon mutagenized potato plants

Diploid potato plants heterozygous for the *P. infestans R1* resistance gene were transformed with an Agrobacterium strain containing a *Ds*-transposon T-DNA construct shown in Figure 1 (Pereira *et al*., 1992; El -Kharbotly *et al*., 1995). Transformant Ds416 contained *a Ds* T-DNA insertion on chromosome 5 (El-Kharbotly *et al*., 1996a), linked in repulsion phase to the previously mapped *P. infestans R1* resistance gene (Leonards-Schippers *et al*., 1992). This Ds416 clone was crossed to the susceptible diploid genotypes J89-5040-2 producing offspring that enabled the selection of recombinant plants (Ds53-22 and -34) having the *R1* gene and the *Ds* T-DNA in coupling phase (18 cM) (El-Kharbotly *et al.,* 1996a). To activate the *Ds* transposon these plants were crossed with TM17-2, a diploid potato clone susceptible to *P*. *infestans* and transformed with the *Ac* transposon-containing T-DNA construct pMKIGBSS*Ac* (Pereira *et al*., 1991). TM17-2 contained one functional *Ac* displaying active transposition. From the progeny of these crosses, population EE96-4311 (Ds53-22 X TM17-2; 18 seedlings) and EE96-4312 (Ds53-34 X TM17-2; 96 seedlings), 47 (8 and 39) kanamycin resistant *R1* seedlings (KanR *R1*) were selected.

Plant genomic DNA was isolated from greenhouse grown leaves (Pereira and Aarts, 1998) and used for molecular analysis. Empty donor sites (EDS-PCR) indicating excision were detected in 22 of the 47 KanR *R1* seedlings as a 450-bp PCR product using specific primers (Figure 1) and confirmed by Southern blot analysis. After selection of the 22 *R1* resistant seedlings showing active *Ds* excision (R1Ds/r-; Ac/-), the expression of hygromycin resistance (HygR) was tested by rooting on MS30 supplemented with 10-100-mg/l hygromycin. One genotype EE96-4311-12 showed resistance by rooting on 40 mg/l hygromycin and displayed a clear EDS fragment, suggesting that screening for rooting of shoots on 40 mg/l could be used as a stringent criteria for *Ds* excision.

As most genotypes contained excision events that occurred late in shoot development these HygR cells could be selected by protoplast isolation and screening for hygromycin resistance to select independent excision events. Protoplasts were isolated from 4-week-old *in vitro* grown shoots (Uijtewaal *et al,* 1987), re-suspended in culture medium TM2G (Wolters *et al*., 1991) to a final concentration of 500,000 pp/ml and diluted weekly with fresh medium. The regenerating calli were progressively transferred to callus growth medium, shoot induction medium and finally maintained on shoot elongation medium until regenerated plants could be harvested (Mattheij *et al*., 1992). In separate experiments to select specifically for protoplast regenerants with excision events, 10 mg/l hygromycin was added to the callus growth medium 14 days after protoplast isolation, then increased to 20 mg/l on day 21 and maintained at this level.

Table 1 gives an overview of protoplast regeneration data. From parental clone Ds53-34, control EE96-4312-21 and selected R1Ds/r-; Ac/- seedlings about 50 regenerating shoots were tested for their rooting ability on MS30 with 40 mg/l hygromycin. As expected, the parent Ds53-34 and control EE96-4312-21 produced no HygR protoplast regenerants whereas EE96-4311-12 gave 45% HygR protoplast regenerants confirming early excision. The other 14 good performing R1Ds/r-; Ac/- plants showed regeneration of 4 to 33% of HygR shoots indicating excision of *Ds* from its original T-DNA location. The use of hygromycin selection during callus culture and regeneration of shoots increased the recovery of HygR regenerants 3.8 times. A total of 1973 HygR regenerants were obtained from different selection experiments and transferred to the greenhouse.

**Table 1:**

| Selection of excision events after protoplast regeneration with and without hygromycin selection. Number of calli, shoots and selected hygromycin resistant (HygR) regenerants for parents Ds53-22 and Ds53-34; control EE96-4312-21 (R1Ds/r-; -/-) and 22-selected R1Ds/r-; Ac/- genotypes from the seedling populations EE96-4311 and EE96-4312. ^{a} low due to infection. | | | | | | |
|---|---|---|---|---|---|---|
| Genotype | No selection During protoplast regeneration | | | Hygromycin selection During protoplast regeneration | | |
| | Calli | Shoots | HygR | Calli | Shoots | HygR |
| Ds53-22 | 10 | 0 | | 47 | 0 | |
| Ds53-34 | 100 | 45 | 0 | 134 | 1 | 0 |
| EE96-4312-21 | 100 | 21 | 0 | 900 | 10 | 0 |
| | | | | | | |
| EE96-4311-08 | 0^{a} | | | 0^{a} | | |
| EE96-4311-12 | 100 | 49 | 22 | 1000 | 198 | 98 |
| EE96-4311-15 | 300 | 82 | 11 | 800 | 160 | 101 |
| | | | | | | |
| EE96-4312-03 | 100 | 23 | 3 | 1000 | 166 | 83 |
| EE96-4312-05 | 100 | 29 | 8 | 1000 | 198 | 121 |
| EE96-4312-06 | 100 | 6^{a} | 2 | 1000 | 205 | 139 |
| EE96-4312-14 | 100 | 70 | 15 | 1000 | 208 | 118 |
| EE96-4312-23 | 100 | 51 | 2 | 1000 | 211 | 91 |
| EE96-4312-27 | 10 | 0 | | 10 | 0 | |
| EE96-4312-28 | 100 | 47 | 7 | 1000 | 143 | 82 |
| EE96-4312-30 | 100 | 0 | | 419 | 0 | |
| EE96-4312-31 | 100 | 30 | 2 | 570 | 21 | 4 |
| EE96-4312-37 | 100 | 52 | 2 | 1000 | 248 | 92 |
| EE96-4312-40 | 33 | 2 | 0 | 67 | 0 | |
| EE96-4312-43 | 100 | 45 | 8 | 650 | 207 | 101 |
| EE96-4312-46 | 14 | 9 | 0 | 103 | 0 | |
| EE96-4312-49 | 100 | 48 | 3 | 1000 | 206 | 109 |
| EE96-4312-52 | 3 | 3 | 0 | 1 | 1 | 1 |
| EE96-4312-60 | 100 | 52 | 7 | 1000 | 203 | 93 |
| EE96-4312-63 | 100 | 50 | 7 | 1000 | 203 | 130 |
| EE96-4312-76 | 0 | 0 | | 24 | 0 | |
| EE96-4312-89 | 100 | 49 | 4 | 274 | 41 | 19 |
| Total | | 691 | 103 | | 2619 | 1382 |

To analyze *Ds* excision in the HygR protoplast regenerants Southern blot hybridization was performed on a subset of selected R1Ds/r-; Ac/- seedlings and some of their HygR protoplast regenerants (Figure 2). Plant DNA was restricted with *Pst*1 and the blots hybridized to probes derived from the NOS promoter fragment that revealed the *Ac* T-DNA and the *Ds* transposon. The R1Ds/r-; Ac/- seedlings used for protoplast isolation all displayed two *Pst1* fragments, respectively 4.0- and 3.5-kb, corresponding to respectively the *Ds* T-DNA and the *Ac* T-DNA constructs. Faintly visible fragments of 2.3-kb were also detected that correspond to a low amount of EDS fragments present in these seedlings. All HygR protoplast regenerants showed a strong hybridizing EDS fragment indicating early or repeated excision *of Ds* corresponding to the high level of hygromycin resistance for which these plants were selected. The original *Ds* parent had two copies of *Ds* at one locus. Full donor site fragments were detected in most of the HygR protoplast regenerants which indicates that one of the two *Ds*'s was not excised. Three plants shown in Fig 2, showed a complete EDS indicating that excision occurred in the initial protoplast. Most HygR regenerants showed clear *Ds* re-insertion fragments varying from 1 to 8 new positions per individual HygR regenerant. Regenerants from a single seedling showed different re-insertion patterns, indicating that they originated from independent transposition events and confirmed that most selected HygR regenerants originate from independent transposition events.

The somatic selection *of Ds* transpositions from individual cells facilitated the production of a large population of shoots with independent *Ds* excision events. The HygR protoplast regenerants potentially represent about 2000 independent *Ds* insertions. This number *of Ds* insertion mutations should be enough for the isolation of tagged mutants involved in *R1* resistance. The somatic selection *of Ds* transposition and the rapid production of independent plants containing these transpositions, facilitates the production of large tagging populations needed for the transposon mutagenesis of selected genes. This is particularly suitable for the mutagenesis of genes in heterozygous crops like potato.

### Screen for R1 type HR resistance variants in the Ds tagged population

The transposon mutagenized population was suitable for the isolation of mutations in defense related genes causing an altered reaction to *P. infestans*. By using a suitable screen quantitative changes towards susceptibility were possible to be identified. Race specific resistance *Cf* genes in tomato have shown a semidominant phenotype if screened in a quantitative manner (Hammond-Kosack and Jones, 1994). Chromosome 5 in potato is known to contain many resistance components (Leonards-Schippers *et al*., 1994) that are probably in a heterozygous state as seen from segregation of minor factors. These loci could probably be efficiently mutagenized due to active linked transposition of *Ds* near *R1*.

To prepare the inoculum for screening (El-Kharbotly *et al*., 1994), *P. infestans* race 0 (89148-09) was grown on rye agar medium (with 20 mg/l sucrose). The sporangiospores were washed with 10-15 ml cold tap water (4°C) and the resulting suspension used to inoculate 10 Bintje tuber slices (1 cm thickness). The newly formed sporangiospores were washed and again used to inoculate 20-50 tuber slices of Bintje in order to obtain 1-2 1 of sporangiospore solution. This solution was diluted to contain at least 2000 spores/ml to use for plant inoculation.

The 1973 hygromycin resistant protoplast regenerant (HRPR's) plants to be tested were periodically brought in batches to the greenhouse. After 6-10 weeks growth two leaves of each HRPR plant were harvested, placed in columns of water absorbent substrate, and put in containers (46 x 31 x 8 cm) closed with transparent covers. In every container two leaves of 10 HRPR plants and a leaf of the susceptible control (Bintje or TM17-2) were tested. In each experiment 15-30 containers were used so that 150-300 plants could be tested in parallel, with Ds53-22, Ds53-34 and TM17-2 always tested as additional controls. Each leaf in the experiments was sprayed with about 5-10 ml of the sporangiospore solution containing 10,000-50,000 sporangiospores. After 5 days in high humidity at 16°C, all leaves were evaluated for the development of *P. infestans* infection symptoms and at day 6 a second evaluation for disease symptoms was performed. When development of symptoms occurred the leaves were kept for an additional 2 days for a microscopic examination of the disease development.

The susceptible parent and control cultivar Bintje always showed distinctive colonization and abundant sporulation on day 5-6 (Figure 3a and 3b). In contrast the resistant parents Ds53-22, Ds53-34 and most of the analyzed HRPR's always displayed characteristic *R1* type HR spots upon infection. The phenotype of HRPR 836 was distinctly susceptible with colonization and sporulation over large leaf areas (Figure 3c). Other HRPR's sometimes showed larger necrotic regions indicating colonization of the leaves (Figure 3d). When this colonization resulted in sporulation (Figure 3f) the HRPR was scored as a potentially susceptible *R1* variant, although necrotic spots were additionally visible on the green parts (Figure 3e) indicating at least a partial HR activation. In this first round of screening 33 putative susceptible variants, derived from 10 R1Ds/r-; Ac/- seedlings were selected (Table 2). Re-inoculation tests of newly grown leaves of the selected variants confirmed the susceptible reactions for 9 variants.

**Table 2:**

| Primary screen for mutants with an altered *R1* type HR resistance response. | | | | | |
|---|---|---|---|---|---|
| R1Ds/r-;Ac/Seedling | total# selected HRPR's | # HRPR's tested with *P. infestans* race 0 | # HRPR's Variant | Variant plant # | Ploidy level |
| EE96-4311-12 | 126 | 72 | 1 | 702 | 4x |
| EE96-4311-15 | 112 | 81 | 2 | 35 | 2x |
| | | | | 994 | 2x |
| EE96-4312-03 | 86 | 63 | 2 | 1515* | 2x |
| | | | | 1921 | nd |
| EE96-4312-05 | 129 | 84 | 2 | 836* | 4x |
| | | | | 842 | 4x |
| EE96-4312-06 | 243 | 188 | | | |
| EE96-4312-14 | 195 | 168 | 2 | 925 | 4x |
| | | | | 1587 | 2x |
| EE96-4312-23 | 93 | 82 | | | |
| EE96-4312-27 | 5 | 0 | | | |
| EE96-4312-28 | 89 | 71 | 7 | 487* | 2x |
| | | | | 998 | 2x |
| | | | | 999 | 4x |
| | | | | 1000 | 2x |
| | | | | 1001 | 4x |
| | | | | 1005 | 4x |
| | | | | 1357 | 2x |
| EE96-4312-31 | 7 | 4 | | | |
| EE96-4312-37 | 134 | 120 | 4 | 151* | 4x |
| | | | | 510* | 4x |
| | | | | 524 | 2x |
| | | | | 551 | 4x |
| EE96-4312-43 | 109 | 91 | 6 | 570* | 4x |
| | | | | 688* | 4x |
| | | | | 1528 | 4x |
| | | | | 561 | x-4x |
| | | | | 562 | x-4x |
| | | | | 574 | 4x |
| EE96-4312-49 | 152 | 111 | 6 | 600 | 4x |
| | | | | 601 | 4x |
| | | | | 633* | 2x |
| | | | | 1050 | 2x |
| | | | | 1055* | 4x |
| | | | | 1073 | 4x |
| EE96-4312-52 | 1 | 1 | | | |
| EE96-4312-60 | 134 | 112 | 1 | 667 | 4x |
| EE96-4312-63 | 168 | 155 | | | |
| EE96-4312-76 | 83 | 65 | | | |
| EE96-4312-89 | 107 | 96 | | | |
| Total | 1973 | 1564 | 33 | | |

| | | | | | |
|---|---|---|---|---|---|
| # = number, nd = not determined, | | | | | |
| * variants with confirmed susceptible reaction after re-inoculation. | | | | | |

Selected genotypes were transferred from the greenhouse to *in vitro* for propagation to obtain 10 or 35 cuttings of each variant and these were transferred again to the greenhouse for a replicated retesting of the *P. infestans R1* resistance. From the first set of 33 *R1* variants, ploidy level analysis enabled the identification of plants with chromosomal anomalies that were potentially somaclonal variants. All the diploid variants together with those with a reproducible susceptible phenotype and the corresponding 9 parental seedlings were used for a secondary quantitative phenotypic analysis. After *P. infestans* inoculation on two leaves of each plant, the developing symptoms were carefully evaluated and followed microscopically when necessary to detect sporulation (Table 3).

**Table 3:**

| Qualitative and quantitative analysis of the resistance reaction and disease development on selected variants compared to parental controls. | | | | | | | |
|---|---|---|---|---|---|---|---|
| | | # plants | % leaves with HR | % leaves with <20% necrosis and sporulation | % leaves with 20-100% necrosis, colonization and sporulation (max % leaf area covered) | | % Dead (Rotten) |
| Parents | | | | | | | |
| Ds53-34 | | 10 | 100 | 0 | 0 | | 0 |
| TM17-2 | | 10 | 0 | 0 | 100 | (100) | 0 |

| R1Ds/r-; Ac/- Seedlings | | | | | | | |
|---|---|---|---|---|---|---|---|
| EE96-4312-76 | | 10 | 100 | 0 | 0 | | 0 |
| EE96-4312-43 | | 9 | 94 | 6 | 0 | | 0 |
| EE96-4312-37 | | 19 | 87 | 13 | 0 | | 0 |
| EE96-4312-28 | | 20 | 75 | 23 | 2 | (35) | 0 |
| EE96-4312-49 | | 10 | 75 | 20 | 5 | (25) | 0 |
| EE96-4312-05 | | 20 | 75 | 15 | 5 | (40) | 5 |
| EE96-4312-03 | | 21 | 71 | 19 | 10 | (60) | 0 |
| EE96-4311-15 | | 20 | 60 | 35 | 5 | (50) | 0 |
| EE96-4312-14 | | 19 | 47 | 18 | 32 | (100) | 3 |

| Variants | | | | | | | |
|---|---|---|---|---|---|---|---|
| EE96-4312-43 | 570 | 9 | 100 | 0 | 0 | | 0 |
| | 688 | 7 | 100 | 0 | 0 | | 0 |
| EE96-4312-37 | 510 | 10 | 95 | 5 | 0 | | 0 |
| | 524 | 10 | 90 | 5 | 0 | | 5 |
| EE96-4312-28 | 487 | 35 | 41 | 43 | 16 | (100) | 0 |
| | 998 | 34 | 60 | 38 | 2 | (35) | 0 |
| | 1000 | 35 | 16 | 34 | 50 | (100) | 0 |
| | 1357 | 34 | 63 | 24 | 9 | (70) | 4 |
| EE96-4312-49 | 601 | 10 | 60 | 30 | 10 | (60) | 0 |
| | 633 | 10 | 70 | 25 | 5 | (60) | 0 |
| | 1050 | 9 | 78 | 17 | 5 | (45) | 0 |
| | 1055 | 10 | 80 | 10 | 5 | (50) | 5 |
| EE96-4312-05 | 836 | 34 | 7 | 12 | 68 | (100) | 13 |
| EE96-4312-03 | 1515 | 31 | 76 | 16 | 8 | (50) | 0 |
| EE96-4311-15 | 35 | 14 | 82 | 0 | 7 | (50) | 11 |
| | 994 | 8 | 44 | 0 | 56 | (100) | 0 |
| EE96-4312-14 | 1587 | 1 | 50 | 0 | 50 | (60) | 0 |

| | | | | | | | |
|---|---|---|---|---|---|---|---|
| # = number, HR = hypersensitive response | | | | | | | |

The *R1* resistant parental plant Ds53-34 always showed the complete *R1* type HR response, with small necrotic spots on inoculated leaves. The *R1* resistant progeny of Ds53-22 and Ds53-34 (EE96-4311-15 and EE96-4312-03, 05, 14, 28, 37, 43, 49 and 76) displayed an intermediate resistance phenotype (Table 3). With the exception of seedling EE96-4312-76, all other seedlings showed on several leaves (6-35%) necrotic spots that developed into necrotic regions covering 5 to 20% of the leaf area (Figure 3g). Microscopic examination revealed very little sporulation in these regions indicating minor escape of *P*. *infestans* from the normal *R1* type HR response. In a few leaves the necrotic region covered almost 100% of the leaf area and colonization with sporulation was observed indicating susceptibility of the leaf and escape from the *R1* type HR resistance response. Seedling EE96-4312-14 showed in this analysis only in 47% of the leaves a clear *R1* type HR response. However, from the 168 HRPR's derived from this seedling and tested in the first screening for *R1* resistances only 2 were selected as putative variants (Table 2). This indicates that the intermediate phenotype for this and other parental seedlings did not result in an overestimation of putative variants in the first screening.

The re-evaluation of the resistance response reaction for the variants 487, 1000, 836 and 994 showed a clear deviation in phenotype when compared to the parental seedlings. Variant 1000 showed colonization and sporulation on 50% of the leaves and this clearly resembled the TM17-2 *P. infestans* susceptible parental phenotype (Figure 3h and 3i). Only 16% of the variant 1000 leaves showed the normal *R1* type HR resistance response. In variant 487 the *R1* type HR resistance response was clearly detected in only 41% of the inoculated leaves. On 16% of the leaves necrotic regions covered over 20% of the leaf area and colonization and sporulation was detected indicating a weak susceptible *R1* variant.

The susceptible phenotype of variant 836 in the first screening of the HRPR population (Figure 3c) was repeatable in this analyses but quick senescence of the leaves, resulting in softening and rotting, suggested other causes for the observed susceptible phenotype. Variant 994 showed a striking phenotype as in every plant the youngest leaf showed colonization with sporulation, combined with leaf softening and rotting. The second oldest leaf analyzed always showed a normal *R1* type HR resistance phenotype. The variants 836 and 994 therefore displayed a more susceptible reaction due to interaction with early senescence and were therefore not considered as variants in the expression of *R1* type resistance. Re-evaluation of the resistance phenotype of the variants 570, 688, 510, 524, 633, 1050, 1055, 1515 and 35 did not reveal any quantitative difference when compared to the parental seedlings and were not regarded as mutants in the *R1* resistance reaction.

### Molecular analysis of the tagged mutants

To examine the causal relationship between the *Ds* insertion sites and the observed phenotype mutant 1000 was characterized by Southern blot hybridization. Genomic DNA from appropriate genotypes was restricted with *Hin*dIII and the blots hybridized to a 5' *Ac* probe to determine the presence and positions of the *Ac* and *Ds* elements in the different genotypes. Since *Ds* is derived from *Ac,* the 5'*Ac* probe also identified the *Ds* element (Pereira *et al,* 1992). Additional hybridization of the same blots with an internal *Ac* probe revealed the presence and position of *Ac.* In the different genotypes the *Ds* and *Ac* insertions were identified by specific *Hin*dIII fragments (Figure 4a and 4b). New positions of the *Ds* elements confirmed that both mutants (from same seedling parent) were derived from different *Ds* transposition events in EE96-4312-28 during protoplast regeneration (Fig 4a). These hybridizations also revealed that mutant 1000 had lost the *Ac* element and was therefore a stable mutant.

To analyze the sites of *Ds* insertion, flanking DNA of *Ds* insertions was isolated by inverse PCR (IPCR ;Triglia *et al*, 1998). Plant genomic DNA, was restricted with *Hae*III, self-ligated and restricted with *Bam*HI and *Bgl*II. *Bgl*II restriction prevents the amplification of the *Ds* transposon flanking sequences in the original T-DNA construct. To obtain additional and longer 5' flanking sequences a second restriction combination was used, in which genomic DNA was restricted with *Msc*I followed by *Hin*dIII and after ligation linearized by *Cla*I. Primer 5'-CGG GAT GAT CCC GTT TCG TT (*Ac* position 197-216) and primer 5'-GAT AAC GGT CGG TAC GGG AT (*Ac* position 44 - 35) were used to amplify the 5' *Ds*/*Ac* flanking sequences. After a hot start (10 min 94°C), 35 PCR cycles (1 min 94°C, 1 min 60°C, 2 min 72°C) resulted in the amplification of *Ac* and *Ds* 5' flanking sequences.

Thermal asymmetric interlaced (TAIL) PCR (Liu and Whittier, 1995) was used to obtain additional *Ds* transposon flanking sequences. Sets of nested primers designed on the 5'- and the 3' site of the *Ac* transposon (Tsugeki *et al*., 1996; Ds5-1, 5-2, 5-3, 5-4 and Ds3-1, 3-2, 3-3, 3-4) were combined with 4 different degenerated primers (AD 1 to 4; Liu and Whittier, 1995) or two other degenerate primers (Tsugeki *et al*., 1996; renamed AD5 and 6). The three step PCR reactions were performed as described (Tsugeki *et al*., 1996). Primers AD3, AD5 and AD6 with *Ac*/*Ds* 5' primers and primers AD5 and AD6 with *Ac*/*Ds* 3' primers produced specific PCR fragments.

The IPCR and TAIL-PCR products were separated on a 1% TBE agarose gel to determine the number and size of fragments. After phenol:chloroform extraction and isopropanol precipitation, the PCR products were cloned in pGEM T easy vector (Promega Corporation). For each sample three clones were sequenced using an automated ABI 373 DNA sequencer. The obtained *Ds* flanking sequences were compared to known sequences by BlastN and BlastX homology searches (Altschul *et al*., 1997) in the public databases.

In mutant 1000 two different *Ds* insertions were identified by BlastX searches, each with homology to a leucine rich repeat containing protein kinase from *Oryza longistaminata* (Tarchini *et al*., 2000) and a receptor protein kinase-like protein from *Arabidopsis thaliana* (BAC clone F13I12). These sequences were both identified due to their homology to the serine/threonine kinase domain of the *Xanthomonas* resistance gene *Xa21* isolated from *O. longistaminata* (Song *et al.,* 1995). Additional *Ds* flanking sequences were isolated using TAIL-PCR (540 bp; Fig 5a). Combining the 5' 288 bp and the 3' 540 bp flanking sequence for this *Ds* insertion revealed the expected 8-bp target site duplication. The sequence flanking this *Ds* insertion showed 50% protein identity to XA21. For the second *Ds* insertions the IPCR and TAIL-PCR only extended the 5' flanking sequence from 496 to 1309 bp (Fig 5a). This sequence covered a complete serine/threonine protein kinase ORF (nucleotides 20 to 583 and 727 to 1053) with 44% identity to the serine/threonine protein kinase domain of XA21 including the conserved intron position , nucleotides 584 to 726 (Fig 5a). All eleven protein kinase specific domains with conserved features were present (Fig 5b) as well as all the 14 conserved amino acids (Hanks *et al*., 1988). Domain VI (consensus DLKPEN) and domain VIII (consensus G(T/S)XX(Y/F)XAPE) are indicative of serine/threonine specificity (Hanks *et al*., 1988). The two *Ds* insertion loci displayed 84% identity at the protein level while in the intron region they showed only 52% nucleotide identity. This was a clear indication that the isolated *Ds* flanking *Solanum tuberosum* protein kinase (StPK) represented two distinct *Ds* tagged loci in mutant 1000, StPK-A and StPK-B. Fig. 6(A) shows the nucleotide sequence of StPK-B (SEQ ID NO: 1) and Fig. 6(B) shows the deduced amino acid sequence of StPK-B (SEQ ID NO: 2).

Although the protein identity was less than 50%, all characteristic protein kinase domains and conserved amino acids were present in the potato insertion loci StPK-A and StPK-B (except for domain X and XI in StPK-A), including the intron position at exactly the same position in the serine/threonine specific domain VIII. Therefore, it is probable that these serine/threonine protein kinases are similarly functional in the signal transduction pathway leading to *P*. *infestans* resistance and perhaps other pathogens.

Surprisingly, the StPKs are more homologous to the protein kinase domain of the rice resistance gene *Xa21* than to the earlier identified *Solanaceous* tomato resistance genes *Pto* (Martin *et al*., 1993) and *Pti* (Zhou *et al*., 1995). More surprisingly, since a homologue of *Pto* was mapped to the *R1* chromosomal 5 area (Leister *et al*., 1996). These tomato serine/threonine kinases are functional in the signal transduction pathway leading to a hypersensitive response reaction upon infection with *Pseudomonas syringae* pv. tomato strains expressing the avirulence gene *avrPto* (Zhou *et al*., 1997). In *Xa21*, other rice homologs (Tarchini *et al*., 2000) and in the StPK-A and StPK-B the conserved intron position in domain VIII indicates a conserved gene family among monocots and dicots. No intron position was identified in the tomato serine/threonine kinases *Pto* and *Pti1.* Among the 11 kinase specific domains only minor differences were observed between the potato kinases and *Xa21* on one hand and *Pto* and *Pti1* on the other hand (Fig 5b). But overall amino acid homology determined that the potato sequences were more related to *Xa21* than to the tomato kinases *Pto* and *Pti1.* Domain IV, with no general consensus, showed a high homology between *Xa21* and the potato sequences while *Pto* and *Pti1* contained different amino acids in this area. Whether this difference determines a clear difference in function or signaling pathway for these kinases needs to be studied.

### Identification of potato protein kinase (StPK) homologs

To characterize the structure of the StPK homologs in *R1* resistant and susceptible plants several sets of primers were designed and used in PCR analysis. Primers EE1 and EE2 (Fig 5a) could amplify a product of expected size of about 470 bp and a second product of about 370 bp from the *R1* resistant parent J91-6167-2, the susceptible parent 87-1024-2 and several *R1* resistant and susceptible progeny (J92-6400-A1, -A2, -A3, -A4, -A5 and -A6). Sequencing the PCR products derived from J91-6167-2, 87-1024-2, J92-6400-A1 and -A4 identified 10 different StPKs (Table 4). In Fig. 7 the DNA sequences of the StPK homologs are shown (partial sequences of the PCR products), SEQ ID NO:3-12. StPK-A was not identified in any of the plants by using this primer combination. From the susceptible parental clone 87-1024-2, StPK-B was isolated. Two additional StPK homologs, StPK-C and -D were identified several times in both *R1* resistant and susceptible clones. StPK-D was the 370 bp PCR product and had a deletion of 108 bp making it very likely a non-functional StPK. From the *R1* resistant plants two additional StPK homologs, StPK-E and -I were isolated and from the susceptible plants 5 additional homologs were isolated, StPK-F, -G, -H, -J and -K (Table 4). The sequences of StPK-F and StPK-G shown in Fig. 7 are smaller than they actually are due to sequencing problems. The isolation of these many StPK homologs indicated that these serine/threonine protein kinases represent a multigene family in *S. tuberosum*. This was confirmed by DNA hybridization, since StPK-B identified over 30 hybridizing fragments in *Hin*dIII or *Eco*RI digested genomic DNA of a single resistant or susceptible potato clone.

**Table 4:**

| Homologs of *Solanum tuberosum* protein kinases (StPK) isolated from *R1* resistant and susceptible clones using primers EE1 and EE2 (Fig 5a) | | | | | | |
|---|---|---|---|---|---|---|
| StPK homologue | % identity to StPK-B | R1r parent 6167-2 | rr parent 1024-2 | R1r progeny J92-6400-A4 | rr progeny J92-6400-A1 | Total Clones |
| StPK-B | 100 | | 1 | | | 1 |
| StPK-C | 92 | 2 | | 3 | 2 | 7 |
| StPK-DΔ | 82 | 5 | 3 | 5 | | 13 |
| StPK-E | 91 | | | 1 | | 1 |
| StPK-F | 91 | | 1 | | | 1 |
| StPK-G | 86 | | 1 | | | 1 |
| StPK-H | 90 | | | | 1 | 1 |
| StPK-1 | 89 | 1 | | | | 1 |
| StPK-J | 91 | | 1 | | | 1 |
| StPK-K | 94 | | | | 2 | 2 |
| | | | | | | |
| Total | | 8 | 7 | 9 | 5 | 29 |

| | | | | | | |
|---|---|---|---|---|---|---|
| Δ deletion locus | | | | | | |

A second set of primers, EE3 and EE6 (Fig 5a), of which primer EE6 is located downstream of the second exon of StPK-B, was specific enough to identify StPK-B in all analyzed plants. So, this StPK homologue is present in 87-1024-2 (identified with EE1 and EE2), in J91-6167-2 and in several tested *R1* resistant and susceptible progeny of population J92-6700, including -A16 from which the tagging population was derived. The StPK-B gene is therefore independent of the *R1* locus.

A third set of primers, EE8 and EE10 (Fig 5a), was designed on low homologous regions between StPK-A and StPK-B and specifically identified the StPK-A locus after *Bgl*II digestion of the PCR products. Analyses of all the parental genotypes used in the different crossings identified that StPK-A is present in the susceptible parent 87-1024-2 that was used to produce the starting population from which J92-6400-A16 was selected (El-Kharbotly *et al*., 1995).

Both *Ds* transposon insertions in mutant 1000 are loci that occur solely or also in plants that do not carry the *R1* gene. Therefore, it is very unlikely that StPK-A or StPK-B are the *R1* gene itself. The *Ds* mutagenized StPK loci were designated respectively *rpr-1* and *rpr-2* (Required for *Phytophthora infestans* resistance). Both homologs cover a complete (or almost complete) serine/threonine protein kinase ORF with all conserved characteristics including a conserved intron position. The *Ds* insertions in *Rpr1* and *Rpr2* probably reduce their expression explaining the incomplete *R1* type HR resistance reaction in mutant 1000. Examples of such mutants that can produce a phenotype are given by mutation in one or two genes of a multigene family (Gilliland *et al*., 1998). The mutations may also be semidominant due to a specific structure as described due to transposon or T-DNA insertions or inversions (Bender and Fink, 1995) (English and Jones, 1998) (Stam *et al*., 1998).

If the StPK homologs are similar to the *Xa21* gene structure with an LRR additional to the kinase domain, then in StPK-A the Ds insertion in the serine/threonine kinase, 46 bp upstream of the intron, would probably form a truncated LRR protein without a functional kinase domain. This putative truncated LRR domain could possibly compete with the functional LRR-kinase genes, reducing or delaying the signal transduction to exhibit partial *P. infestans* resistance.

StPK-B contains a *Ds* insertion downstream of a serine/threonine protein kinase. For this insertion *Ds* 5' promoter activity (Rudenko *et al*., 1994) could result in the production of an antisense RNA. Post transciptional gene silencing due to the formed aberrant RNA could result in a reduction of kinase activity making the signaling pathway leading to the *R1* type HR response less effective. This might explain the semi-dominant mutation leading to a mutated *R1* resistance phenotype in regenerant 1000. A delay in HR response could allow escape of the *P. infestans* from necrotic regions resulting in sporulation and further colonization of the infected leaves.

### Transformation of StPK gene constructs conferring resistance

The StPK-B gene fragment was isolated and incorporated in binary vectors for transforming plants. A suitable vector construct with appropriate regulatory sequences including promoter sequences, terminator fragments, polyadenylation sequences, enhancer sequences, marker genes and other appropriate sequences. The StPK-B fragment encodes the kinase domain but lacks the N-terminal part including the translation initiation part. Suitable constructs (I) and (II) are shown in Fig 8. In one construct (I) a complete kinase encoding gene was made with the StPK-B gene consisting of the DNA fragment shown as Seq ID 1, with a translation start coding for the methionine codon in frame with the open reading frame shown in Seq ID 2. In this construct-I the engineered StPK-B gene was cloned in between the constitutive CaMV 35S promoter and a nopaline synthase (Nos) terminator in the vector pBINPLUS (van Engelen et al., 1995). In another construct type (II) a translational fusion made between the STPK-B fragment of SEQ ID land a N-terminal part of the complete gene from STPK-B or homologues genes is made. These gene fusions are cloned in between the appropriate regulatory promoter and terminator sequences in pBINPLUS.

The above mentioned recombinant binary vectors are possible to construct by persons skilled in the art, including the transfer into appropriate Agrobacterium strains and checking for their stable presence in the Agrobacterium. The recombinant Agrobacterium construct containing the StPK-B overexpression cassette are transformed into potato plants by established procedures (El-Kharbotly et al., 1995). A set of transformants are regenerated and multiplied in vitro by cutting. About 5 regenerated plantlets from each individual transformant are transferred to the greenhouse.

At about 6 weeks after transferring to the greenhouse the replicated sample of the transformants are tested by the detached leaf test for *Phytophthora infestans* resistance as described in detail above. From each plant two leaflets are taken, amounting to 10 leaf samples per individual transformant. The resistance score over the replicate samples provides a quantitative estimate of the resistance reaction and allows the selection of plants significantly resistant to infection by Phytophthora.

### References

**Aarts, M.G.M., Dirkse, W.G., Stiekema, W.J. and Pereira, A.** (1993) Transposon tagging of a male sterility gene in *Arabidopsis. Nature* **363**, 715-717.

**Aarts, N., Metz, M., Holub, E., Staskawicz, B.J., Daniels, M.J. and Parker, J.E.** (1998) Different requirements for *EDS1* and *NDR1* by disease resistance genes define at least two *R* gene-mediated signaling pathways in *Arabidopsis, Proceedings of the National Academy of Sciences of the United States of America* **95**, 10306-10311.

**Altschul, S.F., Madden, T.L., Schäffer, A.A., Zhang, J., Zhang, Z., Miller, W. and Lipman, D.J.** (1997) Gapped BLAST and PSI-BLAST: a new generation of protein database search programs. *Nucleic Acids Research* **25,** 3389-3402.

**Baker, B., Coupland, G., Fedoroff, N., Starlinger, P. and Schell, J.** (1987) Phenotypic assay for excision of the maize controlling element, *Ac* in tobacco. *EMBO Journal* **6,** 1547-1554.

**Bender, J. and Fink, G.R.** (1995) Epigenetic control of an endogenous gene family is revealed by a novel blue fluorescent mutant of Arabidopsis. *Cell* **83,** 725-734.

**Century, K.S., Holub, E.B. and Staskawicz, B.J.** (1995) *NDR1*, a locus of *Arabidopsis thaliana* that is required for disease resistance to both a bacterial and a fungal pathogen. *Proceedings of the National Academy of Sciences of the United States of America* **92**, 6597-6601.

**Chuck, G., Robbins, T., Nijjar, C., Ralston, E., Courtney-Gutterson, N. and Dooner, H.K.** (1993) Tagging and cloning of a petunia flower color gene with the maize transposable element *Activator*. *Plant Cell* **5**, 371-378.

**Collins, A., Milbourne, D., Ramsay, L., Meyer, R., Chatot-Balandras, C., Oberhagemann, P., de Jong, W., Gebhardt, C., Bonnel, E. and Waugh, R.** (1999) QTL for field resistance to late blight in potato are strongly correlated with maturity and vigour. *Molecular breeding* **5,** 387-398.

El-Kharbotly, A., Jacobs, J.M.E., te Lintel-Hekkert, B., Jacobsen, E., Ramanna, M.S., Stiekema, **W.J. and Pereira, A.** (1996a) Localization of *Ds*-transposon containing T-DNA inserts in the diploid transgenic potato: linkage to the *R1* resistance gene against *Phytophthora infestans* (Mont.) de Bary. *Genome* **39,** 249-257.

**El-Kharbotly, A., Jacobsen, E., Stiekema, W.J. and Pereira, A.** (1995) Genetic localisation of transformation competence in diploid potato. *Theoretical and Applied Genetics* **91,** 557-562.

**El-Kharbotly, A., Leonards-Schippers, C., Huigen, D.J., Jacobsen, E., Pereira, A., Stiekema, W.J., Salamini, F. and Gebhardt,** C. (1994) Segregation analysis and RFLP mapping of the *R1* and *R3* alleles conferring race-specific resistance to *Phytophthora infestans* in progeny of dihaploid potato parents. *Molecular and General Genetics* **242,** 749-754.

**El-Kharbotly, A., Pereira, A., Stiekema, W.J. and Jacobsen, E.** (1996b) Race specific resistance against *Phytophthora infestans* in potato is controlled by more genetic factors than only *R*-genes. *Euphytica* **90**, 331-336.

**Ellis, J. and Jones, D.** (1998) Structure and function of proteins controlling strain-specific pathogen resistance in plants. *Current opinion in plant biology* **1,** 288-293.

**English, J., J. and Jones, J.D.G.** (1998) Epigenetic instability and trans-silencing interactions associated with an SPT::Ac T-DNA locus in tobacco. *Genetics. Jan*. **148,** 457-469.

**Gilliland, L.U., McKinney, E.C., Asmussen, M.A. and Meagher, R.B.** (1998) Detection of deleterious genotypes in multigenerational studies. I. Disruptions in individual Arabidopsis actin genes. *Genetics* **149,** 717-725.

**Glazebrook, J., Rogers, E.E. and Ausubel, F.M.** (1996) Isolation of Arabidopsis mutants with enhanced disease susceptibility by direct screening. *Genetics* **143,** 973-982.

**Glazebrook, J., Zook, M., Mert, F., Kagan, I., Rogers, E.E., Crute, I.R., Holub, E.B., Hammerschmidt, R. and Ausubel, F.M.** (1997) Phytoalexin-deficient mutants of *Arabidopsis* reveal that *PAD4* encodes a regulatory factor and that four *PAD* genes contribute to downy mildew resistance. *Genetics* **146,** 381-392.

**Graham, K.M.** (1963) Inheritance of resistance to *Phytophthora infestans* in two diploid Mexican Solanum species. *Euphytica* **12,** 35-40.

**Hammond-Kosack, K.E. and Jones, J.D.G.** (1994) Incomplete dominance of tomato *Cf* genes for resistance to *Cladosporium fulvum. Molecular Plant-Microbe Interactions* **7,** 58-70.

**Hammond-Kosack, K.E. and Jones, J.D.G.** (1997) Plant disease resistance genes. *Annual Review of Plant Physiology and Plant Molecular Biology* **48,** 575-607.

**Hanks, S.K., Quinn, A.M. and Hunter, T.** (1988) The protein kinase family: conserved features and deduced phylogeny of the catalytic domains. *Science, USA* **241,** 42-52.

**Innes, R.W.** (1998) Genetic dissection of R gene signal transduction pathways. *Current Opinion in Plant Biology* **1,** 299-304.

**Jia, Y., Loh, Y.T., Zhou, J. and Martin, G.B.** (1997) Alleles of Pto and Fen occur in bacterial speck-susceptible and fenthion-insensitive tomato cultivars and encode active protein kinases. *Plant Cell* 9,61-73.

**Jones, D.A., Thomas, C.M., Hammond-Kosack, K.E., Balint-Kurti, P.J. and Jones, J.D.G.** (1994) Isolation of the tomato *Cf-9* gene for resistance to *Cladosporium fulvum* by transposon tagging. *Science* **266,** 789-793.

**Jones, J.D.G., Romeis, T., Thomas, C., Piedras, P., Durrant, W., Noel, L., Parker, J., Rivas, S., Golstein, C., Dixon, M. and van der Biezen, E.** (1999) Molecular genetics of *Cf*-and *RPP*-disease resistance gene function. In *9th International Congres on Molecular Plant-Microbe Interactions*, Biology of Plant-Microbe Interactions, Volume 2 (Wit, P.J.G.M.d., Bisseling, T. and Stiekema, W.J., eds). Amsterdam: International Society of Molecular Plant-Microbe Interactions, pp. 243-248.

**Jørgensen, J.H.** (1996) Effect of three suppressors on the expression of powdery mildew resistance genes in barley. *Genome* **39**, 492-498.

**Kreike, C.M., de Koning, J.R.A., Vinke, J.H., van Ooijen, J.W. and Stiekema, W.J.** (1994) Quantitatively inherited resistance to *Globodera pallida* is dominant by one major locus in *Solanum spegazzinii. Theoretical and Applied Genetics* **88**, 764-769.

**Lawrence, G.J., Finnegan, E.J., Ayliffe, M.A. and Ellis, J.G.** (1995) The *L6* gene for flax rust resistance is related to the Arabidopsis bacterial resistance gene *RPS2* and the tobacco viral resistance gene *N. Plant Cell* **7**, 1195-1206.

**Leister, R.T., Ausubel, F.M. and Katagiri, F.** (1996) Molecular recognition of pathogen attack occurs inside of plant cells in plant disease resistance specified by the Arabidopsis genes RPS2 and RPMl. *Proceedings of the National Academy of Sciences of the United States of America* **93,** 15497-15502.

**Leonards-Schippers, C., Gieffers, W., Salamini, F. and Gebhardt, C.** (1992) The *R1* gene conferring race-specific resistance to *Phytophthora infestans* in potato is located on potato chromosome V. *Molecular and General Genetics* **233**, 278-283.

**Leonards-Schippers, C., Gieffers, W., Schäfer-Pregl, R., Ritter, E., Knapp, S.J., Salamini, F. and Gebhardt, C.** (1994) Quantitative resistance to *Phytophthora infestans* in potato: a case study for QTL mapping in an allogamous plant species. *Genetics* **137,** 67-77.

**Liu, Y.G. and Whittier, R.F.** (1995) Thermal Asymmetric Interlaced PCR: Automatable Amplification and Sequencing of Insert End Fragments from P1 and YAC Clones to Chromosome Walking. *Genomics* **25,** 674-681.

**Martin, G.B., Brommonschenkel, S.H., Chunwongse, J., Frary, A., Ganal, M.W., Spivey, R., Wu, T.Y., Earle, E.D. and Tanksley, S.D.** (1993) Map-based cloning of a protein kinase gene conferring disease resistance in tomato. *Science Washington* **262,** 1432-1436.

**Mattheij, W.M., Eijlander, R., de Koning, J.R.A. and Louwes, K.M.** (1992) Interspecific hybridization between the cultivated potato *Solanum tuberosum* ssp. *tuberosum* L. and the wild species *Solanum circaeifolium* ssp *circaeifolium* Bitter exhibiting resistance to *Phytophthora infestans* (Mont.) de Bary and *Globodera pallida* (Stone) Behrens: 1. Somatic hybrids. *Theoretical and Applied Genetics* **83,** 459-466.

**McDowell, J.M., Cuzick, A., Can, C., Beynon, J., Dangl, J.L. and Holub, E.B.** (2000) Downy mildew (Peronospora parasitica) resistance genes in Arabidopsis vary in functional requirements for NDR1, EDS1, NPR1 and salicylic acid accumulation. *Plant journal* **22,** 523-529.

**Parker, J.E., Holub, E.B., Frost, L.N., Falk, A., Gunn, N.D. and Daniels, M.J.** (1996) Characterization of *eds1,* a mutation in Arabidopsis suppressing resistance to *Peronospora parasitica* specified by several different *RPP* genes. *Plant Cell* **8,** 2033-2046.

**Parniske, M., Hammond-Kosack, K.E., Golstein, C., Thomas, C.M., Jones, D.A., Harrison, K., Wulff, B.B.H. and Jones, J.D.G.** (1997) Novel disease resistance specificities result from sequence exchange between tandemly repeated genes at the Cf-4/9 locus of tomato. *Cell Cambridge* **91,** 821-832.

**Pereira, A., Aarts, M., van Agtmaal, S., Stiekema, W.J. and Jacobsen, E.** (1991) Waxy variegation in transgenic potato. *Maydica* **36,** 323-327.

**Pereira, A. and Aarts, M.G.M.** (1998) Transposon tagging with the *En-I* system. In *Arabidopsis protocols,* Methods in Molecular Biology, Volume 82 (Martinez-Zapater, J.M. and Salinas, J., eds). Totowa, New York: Humana Press Inc., pp. 329-338.

**Pereira, A., Jacobs, J.M.E., te Lintel-Hekkert, W., Rutgers, E., Jacobsen, E. and Stiekema, W.J.** (1992) Towards the isolation of resistance genes by transposon targeting in potato. *Netherlands Journal of Plant Pathology* **98,** 215-221.

**Ritter, E., Debener, T., Barone, A., Salamini, F. and Gebhardt, C.** (1991) RFLP mapping on potato chromosomes of two genes controlling extreme resistance to potato virus X (PVX). *Molecular and General Genetics* **227,** 81-85.

**Rommens, C.M.T., Haaren, M.J.J.v., Buchel, A.S., Mol, J.N.M., Tunen, A.J.v., Nijkamp, H.J.J., Hille, J., Van Haaren, M.J.J. and Van Tunen, A.J.** (1992) Transactivation of Ds by Ac-transposase gene fusions in tobacco. *Molecular and General Genetics* **231,** 433-441.

**Rouppe van der Voort, J., Lindeman, W., Folkertsma, R, Hutten, R., Overmars, H., van der** **Vossen, E., Jacobsen, E. and Bakker, J.** (1998) A QTL for broad-spectrum resistance to cyst nematode species (*Globodera* spp.) maps to a resistance gene cluster in potato. *Theoretical and Applied genetics* **96,** 654-661.

**Rudenko, G.N., Nijkamp, H.J.J. and Hille, J.** (1994) DS read-out transcription in transgenic tomato plants. *Molecular and General Genetics* **243,** 426-433.

**Salmeron, J.M., Oldroyd, G.E.D., Rommens, C.M.T., Scofield, S.R, Kim, H.-S., Lavelle, D.T., Dahlbeck, D. and Staskawicz, B.J.** (1996) Tomato *Prf* is a member of the leucine-rich repeat class of plant disease resistance genes and lies embedded within the *Pto* kinase gene cluster. *Cell* **86,** 123-133.

**Song, W.-Y., Wang, G.-L., Chen, L.-L., Kim, H.-S., Pi, L.-Y., Holsten, T., Gardner, J., Wang, B., Zhai, W.-X., Zhu, L.-H., Fauquet, C. and Ronald, P.** (1995) A receptor kinase-like protein encoded by the rice disease resistance gene, *Xa21*. *Science* **270,** 1804-1806.

**Stam, M., Viterbo, A., Mol Joseph, N.M. and Kooter Jan, M.** (1998) Position-dependent methylation and transcriptional silencing of transgenes in inverted T-DNA repeats: Implications for posttranscriptional silencing of homologous host genes in plants. *Molecular and Cellular Biology. Nov*. **18**, 6165-6177.

**Tarchini, R., Biddle, P., Wineland, R., Tingey, S. and Rafalski, A.** (2000) The complete sequence of 340 kb of DNA around the rice *Adh1-Adh2* region reveals interrupted colinearity with maize chromosome 4. *Plant Cell* **12,** 381-391.

**Toxopeus, H.J.** (1958) Note on the variation in expression of hypersensitivity to *Phytophthora infestans* in potato seedlings. *Euphytica* **7**, 38-40.

**Triglia, T., Peterson, M.G. and Kemp, D.J.** (1988) A procedure for *in vitro* amplification of DNA segments that lie outside boundaries of known sequences. *Nucleic Acids Research* **16,** 8186.

**Tsugeki, R., Kochieva, E.Z. and Fedoroff, N.V.** (1996) A transposon insertion in the Arabidopsis SSR16 gene causes an embryo-defective lethal mutation. *Plant Journal* **10,** 479-489.

**Uijtewaal, B.A., Suurs, L.C.J.M. and Jacobsen, E.** (1987) Protoplast fusion of monohaploid (2n = x = 12) potato clones: identification of somatic hybrids using malate dehydrogenase as a biochemical marker. *Plant Science* **51,** 277-284.

**Van Engelen, F.A., Molthoff, J.W., Conner, A.J., Nap, J.-P., Pereira A. and Stiekema, W.J.** (1995) pBINPLUS: an improved plant transformation vector based on pBIN19. *Transgenic Res.* **4**, 288-290.

**Warren, R.F., Merritt, P.M., Holub, E. and Innes, R.W.** (1999) Identification of three putative signal transduction genes involved in *R* gene-specified disease resistance in Arabidopsis. *Genetics* **152**,401-412.

**Wastie, R.L.** (1991) Breeding for resistance. In *Phytophthora infestans*, *the cause of late blight in potato*, Advances in Plant Pathology, Volume 7. London: Academic Press, pp. 193-224.

**Whitham, S., Dinesh-Kumar, S.P., Choi, D., Hehl, R., Corr, C. and Baker, B**. (1994) The product of the tobacco mosaic virus resistance gene *N:* similarity to Toll and the Interleukin-1 receptor. *Cell* **78**,1101-1115.

**Wolters, A.M.A., Schoenmakers, H.C.H., van der Meulen-Muisers, J.J.M., van der Knaap, E., Derks, F.H.M., Koornneef, M. and Zelcer, A.** (1991) Limited DNA elimination from the irradiated potato parent in fusion products of albino *Lycopersicon esculentum* and *Solanum tuberosum. Theoretical and Applied Genetics* **83,** 225-232.

**Zhou, J., Loh, Y.-T., Bressan, R.A. and Martin, G.B.** (1995) The tomato gene *Pti1* encodes a serine/threonine kinase that is phosphorylated by Pto and is involved in the hypersensitive response. *Cell Cambridge* **83,** 925-935.

**Zhou, J.M., Tang, X.Y. and Martin, G.B.** (1997) The Pto kinase conferring resistance to tomato bacterial speck disease interacts with proteins that bind a cis-element of pathogenesis-related genes. *EMBO Journal* **16**, 3207-3218.

## Claims

1. An isolated DNA sequence which encodes a protein having the amino acid sequence given in SEQ ID NO: 2 or a functionally homologous protein having an amino acid sequence showing an identity of at least 80% to SEQ ID NO: 2, and which confers *Phytophthora infestans* resistance on plants.

2. The DNA sequence according to claim 1, **characterized in that** the functionally homologous protein has an amino acid sequence showing an identity of at least 85% to SEQ ID NO: 2.

3. The DNA sequence according to claim 1 or 2, **characterized in that** it comprises the nucleotide sequence selected from the group consisting of:
a) the DNA sequence given in SEQ ID NO: 1 and its complementary strand, and
b) DNA sequences hybridizing to the sequences in (a) under stringent hybridization conditions.

4. The DNA sequence according to claim 3, **characterized in that** it comprises the nucleotide sequence of
nucleotides 20 to 1053 of SEQ ID NO: 1.

5. The DNA sequence according to claim 3, **characterized in that** it comprises the nucleotide sequence of
nucleotides 20 to 583 and 727 to 1053 of SEQ ID NO: 1.

6. A protein encoded by the DNA sequence of any of claims 1 to 5.

7. A recombinant vector comprising a DNA sequence under control of an appropriate promoter and regulatory elements for expression in a host cell, wherein the DNA sequence is as defined in any of claims 1 to 5.

8. Use of a DNA sequence of any of claims 1 to 5 or a recombinant vector of claim 7 for the production of a transgenic plant.

9. A host cell transferred with a DNA sequence of any of claims 1 to 5 or a recombinant vector of claim 7.

10. A transgenic host cell according to claim 9 which is a plant cell.

11. A transgenic plant or any part thereof comprising a plant cell according to claim 10.

12. A transgenic seed, a selfed or hybrid progeny or a descendant of a plant according to claim 11, or any part thereof, comprising a cell according to claim 10.

13. A method of conferring *Phytophthora infestans* resistance on a plant, comprising the steps of
i) introducing a DNA sequence of any of claims 1 to 5 or a recombinant vector of claim 7 into a cell of the plant,
ii) regenerating plants from the obtained transgenic cells, and
iii) selecting plants exhibiting *Phytophthora infestans* resistance.

## Patentansprüche

1. Isolierte DNA-Sequenz, die kodiert für ein Protein mit der Aminosäuresequenz gegeben in SEQ ID NO:2 oder ein funktional homologes Protein mit einer Aminosäuresequenz die eine Identität von zumindest 80% zu SEQ 1D NO:2 aufweist, und welche Resistenz gegen *Phytophthora infestans* in Pflanzen vermittelt.

2. DNA-Sequenz nach Anspruch 1, **dadurch gekennzeichnet, daß** das funktional homologe Protein eine Aminosäuresequenz hat, die eine Identität von zumindest 85% zu SEQ ID NO:2 aufweist.

3. DNA-Sequenz nach Anspruch 1 oder 2, **dadurch gekennzeichnet, daß** sie die Nucleotidsequenz, ausgewählt aus der Gruppe bestehend aus:
a) der DNA-Sequenz gegeben in SEQ ID NO:1 und ihrem komplementären Strang, und
b) zu den Sequenzen in (a) unter stringenten Hybridisierungsumständen hybridisierenden DNA-Sequenzen, umfaßt.

4. DNA-Sequenz nach Anspruch 3, **dadurch gekennzeichnet, daß** sie die Nucleotidsequenz der Nucleotiden 20 bis 1053 von SEQ ID NO:1 umfaßt.

5. DNA-Sequenz nach Anspruch 3, **dadurch gekennzeichnet, daß** sie die Nucleotidsequenz der Nucleotiden 20 bis 583 und 727 bis 1053 von SEQ ID NO:1 umfaßt.

6. Protein durch die DNA-Sequenz nach einem der Ansprüche 1 bis 5 kodiert.

7. Rekombinanter Vektor umfassend eine DNA-Sequenz unter Kontrolle eines geeigneten Promoters und Regelelemente für Expression in einer Wirtzelle, worin die DNA-Sequenz ist wie definiert in einem der Ansprüche 1 bis 5.

8. Benutzung einer DNA-Sequenz nach einem der Ansprüche 1 bis 5 oder eines rekombinanten Vektors nach Anspruch 7 für die Produktion einer transgenischen Pflanze.

9. Wirtzelle mit einer DNA-Sequenz nach einem der Ansprüche 1 bis 5 oder einem rekombinanten Vektor nach Anspruch 7 transformiert.

10. Transgenische Wirtzelle nach Anspruch 9, welche eine Pflanzenzelle ist.

11. Transgenische Pflanze oder irgendeines Teil davon, umfassend eine Pflanzenzelle nach Anspruch 10.

12. Transgenischer Samen, selbstbefruchteter oder hybrider Abkömmling oder Nachkomme einer Pflanze nach Anspruch 11 oder ein Teil davon, umfassend eine Zelle nach Anspruch 10.

13. Verfahren zum Vermitteln von Resistentz gegen *Phytophthora infestans* in eine Pflanze, umfassend die Schritte von
i) Einführen einer DNA-Sequenz nach einem der Ansprüche 1 bis 5 oder eines rekombinanten Vektors nach Anspruch 7 in eine Zelle einer Pflanze,
ii) Regenerieren von Pflanzen der erhaltenen transgenischen Zellen, und
iii) Selektieren von Pflanzen die Resistenz gegen *Phytophthora infestans* darstellen.

## Revendications

1. Séquence d'ADN isolée qui code pour une protéine ayant la séquence d'acides aminés donnée dans la SEQ ID n° : 2 ou une protéine fonctionnellement homologue ayant une séquence d'acides aminés présentant une identité d'au moins 80 % à la SEQ ID n° : 2, et qui confère aux plantes une résistance à *Phytophtora infestans.*

2. Séquence d'ADN selon la revendication 1, **caractérisée en ce que** la protéine fonctionnellement homologue a une séquence d'acides aminés présentant une identité d'au moins 85 % à la SEQ ID n° : 2.

3. Séquence d'ADN selon la revendication 1 ou 2, **caractérisée en ce qu'**elle comprend une séquence nucléotidique choisie dans le groupe constitué de :
a) la séquence d'ADN donnée dans la SEQ ID n° : 1 et son brin complémentaire et
b) les séquences d'ADN s'hybridant aux séquences de (a) dans des conditions d'hybridation stringent.

4. Séquence d'ADN selon la revendication 3, **caractérisée en ce qu'**elle comprend la séquence nucléotidique des nucléotides 20 à 1053 de la SEQ ID n° : 1.

5. Séquence d'ADN selon la revendication 3, **caractérisé en ce qu'**elle comprend la séquence nucléotidique des nucléotides 20 à 583 et 727 à 1053 de la SEQ ID n° : 1.

6. Protéine codée par la séquence d'ADN selon l'une quelconque des revendications 1 à 5.

7. Vecteur recombinant comprenant une séquence d'ADN sous le contrôle d'un promoteur et d'éléments régulateurs appropriés pour l'expression dans une cellule hôte, dans lequel la séquence d'ADN est telle que définie selon l'une quelconque des revendications 1 à 5.

8. Utilisation d'une séquence d'ADN selon l'une quelconque des revendications 1 à 5 ou d'un vecteur recombinant selon la revendication 7, pour la production d'une plante transgénique.

9. Cellule hôte transformée avec une séquence d'ADN selon l'une quelconque des revendications 1 à 5 ou un vecteur recombinant selon la revendication 7.

10. Cellule hôte transgénique selon la revendication 9, qui est une cellule de plante.

11. Plante transgénique ou une partie quelconque de celle-ci, comprenant une cellule de plante selon la revendication 10.

12. Semence transgénique, progéniture autofécondée ou hybride ou descendant d'une plante selon la revendication 11 ou une partie quelconque de celles-ci, comprenant une cellule selon la revendication 10.

13. Procédé pour conférer à une plante une résistance *à Phytophtora infestans,* comprenant les étapes de
i) l'introduction d'une séquence d'ADN selon l'une quelconque des revendications 1 à 5 ou d'un vecteur recombinant selon la revendication 7 dans une cellule de la plante
ii) la régénération des plantes à partir des cellules transgéniques obtenues et
iii) la sélection des plantes présentant la résistance à *Phytophtora infestans.*
